# EUROPÄISCHE PATENTSCHRIFT

(11) **EP 1 296 635 B1**
(45) Veröffentlichungstag und Bekanntmachung des Hinweises auf die Patenterteilung: **29.11.2006**
(21) Anmeldenummer: 01956501.9
(22) Anmeldetag: 23.06.2001
(51) Int. Cl.: A61K 8/37, A61K 8/44, A61K 8/67, A61Q 5/02, A61Q 19/00, A61Q 19/10

(54) **STABILISIERTES KOSMETISCHES MITTEL**
STABILISED COSMETIC AGENTS
SUBSTANCES COSMETIQUES STABILISEES

(30) Priorität: 03.07.2000 DE 10031324
(43) Veröffentlichungstag der Anmeldung: 02.04.2003
(73) Patentinhaber: Henkel Kommanditgesellschaft auf Aktien, 40191 Düsseldorf (DE)
(72) Erfinder: SCHELGES, Heike, 47807 Krefeld (DE); SCHOLZ, Wolfhard, 47829 Krefeld (DE)
(86) Internationale Anmeldenummer: PCT/EP2001/007158
(87) Internationale Veröffentlichungsnummer: WO 2002/002058

(56) Entgegenhaltungen:
- EP-A1- 0 347 664
- EP-A1- 0 353 161
- DE-A- 19 802 204
- DE-A1- 19 943 678
- US-A- 4 975 272

## Beschreibung

Die Erfindung betrifft Produkte bestehend aus einem wäßrigen kosmetischen Mittel in transparenten oder semitransparenten Behältern. Die Produkte sind aufgrund des Gehaltes einer speziellen Stabilisator-Mischung besonders lagerstabil und zeichnen sich durch eine verbesserte Farb- und Geruchsstabilität insbesondere gegenüber der Einwirkung von Licht aus.

Kosmetische Mittel zeigen während der Lagerung eine gewisse Tendenz zur Zersetzung. Hierbei kann es sich beispielsweise um eine Phasentrennung bei Emulsionspräparaten handeln, um Veränderungen des Viskositätsverhaltens oder um Farb- und Geruchsveränderungen. Insbesondere Farb- und Geruchsstabilität spielen für die kosmetische Akzeptanz beim Endverbraucher eine bedeutende Rolle. Farbe und Geruch kosmetischer Präparate können sich ändern, wenn die Präparate erhöhten Temperaturen oder Licht ausgesetzt sind, beispielsweise in Schaufensterauslagen. Um die durch Licht bedingten Veränderungen zu reduzieren, werden kosmetische Mittel daher üblicherweise in nicht-transparente, lichtundurchlässige Behälter abgefüllt. Transparente oder semitransparente Produkt-Behälter werden zur Verbesserung der Lichtstabilität oft aus Materialien gefertigt, denen UV-Filtersubstanzen, wie beispielsweise Benzotriazol, zugesetzt werden. Andererseits können die kosmetischen Mitteln selbst UV-Filtersubstanzen enthalten.

Aus der DE 197 50 906 und der DE 197 39 797 ist beispielsweise der Einsatz von Triazin-Derivaten zur Stabilisierung organischer Materialien gegen UV-Licht, Sauerstoff und erhöhte Temperaturen bekannt. Die EP 0 714 880 betrifft Bismethylenphenylen-Derivate, die nicht nur als Sonnenschutzfaktoren zum Schutz der Haut und Haare Einsatz finden, sondern auch der Verbesserung der Lagerstabilität kosmetischer Zusammensetzungen dienen. Aus den japanischen Offenlegungsschriften JP 09078085 A und JP 10237488 A sind Haut- und Haarreinigungsmittel bekannt, denen zur Verbesserung der Lagerstabilität bei erhöhter Temperatur Komplexbildner und Antioxidantien zugesetzt werden. Stabilisator-Mischungen aus Antioxidantien und Komplexbildnern werden häufig auch zur Stabilisierung retinoidhaltiger kosmetischer Präparate verwendet, wie beispielsweise in der EP 0 440 398, WO 96/07396, EP 0 549 592 und EP 0 586 106 beschrieben.

Das Patentdokument DE-A-19802204 offenbart im Beispiel 9 eine Zusammensetzung enthaltend Tocopherylacetat, NaEDTA und 2.4% Glycerylstearatcitrat als Komponente c.

Der gegenwärtige Verbrauchertrend geht zu kosmetischen Produkten in transparenten und semitransparenten Behältern. Aufgabe war es daher, ein Stabilisatorsystem zu entwickeln, das kosmetische Produkte in transparenten oder semitransparenten Behältern gegen die durch Licht bedingten Farb- und Geruchsveränderungen stabilisiert. Die gegenwärtigen Mittel sind in dieser Hinsicht nicht ausreichend wirksam. Ein besonderer Aspekt der Aufgabe bestand daher darin, die kosmetischen Mittel in derartigen Behältern gegen lichtbedingte Veränderungen stärker zu stabilisieren als dies durch zugesetzte UV-Filtersubstanzen erreicht wird. Ein weiterer Teilaspekt der Aufgabe bestand darin, ein besonders hautfreundliches Stabilisatorsystem zu entwickeln, welches mit einer Vielzahl kosmetischer Formulierungen kompatibel ist und insbesondere tensidhaltige Formulierungen in transparenten oder semitransparenten Behältern auch ohne den Einsatz von UV-Filtem gegen Lichteinwirkung stabilisiert.

Es wurde nun überraschenderweise gefunden, daß sich kosmetische Mittel durch eine bestimmte Stabilisator-Kombination in transparenten oder semitransparenten Produktbehältern gegen lichtbedingte Farb- und Geruchsveränderungen stabilisieren lassen und daher über lange Zeit konstante Produkteigenschaften aufweisen.

Gegenstand der Erfindung sind daher kosmetische Produkte, bestehend aus einem wasserhaltigen kosmetischen Mittel und einem transparenten oder semitransparenten Behälter, dadurch gekennzeichnet, daß das kosmetische Mittel eine Stabilisator-Mischung aus wenigstens einem Tocopherol und/oder Tocopherol-Derivat (A) und b) wenigstens einem Komplexbildner (B) und c) 0.005 bis 1 Gew.% eines Esters (C) aus einer hydroxysubstituierten Bi- oder Tricarbonsäure und einem C₁₂-C₂₄-Fettsäuremono- und/oder -diglycerid enthält.

Ein weiterer Gegenstand der Erfindung ist die Verwendung einer Stabilisator-Mischung aus wenigstens einem Tocopherol und/oder Tocopherol-Derivat (A) und wenigstens einem Komplexbildner (B) und wenigstens einem Ester (C) aus einer hydroxysubstituierten Bi- oder Tricarbonsäure und einem C₁₂-C₂₄-Fettsäuremono- und/oder -diglycerid zur Farb- und Geruchsstabilisierung kosmetischer Mittel in transparenten oder semitransparenten Produktbehältern sowie ein Verfahren zur Farb- und Geruchsstabilisierung kosmetischer Mittel in transparenten oder semitransparenten Behältern, insbesondere gegenüber der Einwirkung von Licht, dadurch gekennzeichnet, daß man den Mitteln eine Stabilisator-Mischung aus wenigstens einem Tocopherol und/oder Tocopherol-Derivat (A) und wenigstens einem Komplexbildner (B) und wenigstens einem Ester (C) aus einer hydroxysubstituierten Bi- oder Tricarbonsäure und einem C₁₂-C₂₄-Fettsäuremono- und/ oder -diglycerid zusetzt.

Die Kombination der drei Komponenten A + B + C führt zu einer außerordentlichen Farb- und Geruchsstabilisierung von wasserhaltigen kosmetischen Mitteln, die in transparente oder semitransparente Behälter abgefüllt sind, die beispielsweise aus Polyethylen, Polypropylen, Polyethylenterephthalat-Glycol, Polycarbonat, Polystyrol oder Glas gefertigt sind. Lagertests haben gezeigt, daß die Stabilisator-Mischung die Geruchs- und Farbstabilität bei Einwirkung von Licht deutlich stärker verbessert als UV-Filtersubstanzen, die im Behältermaterial oder im Produkt enthalten sind.

Die Stabilisatorkombination kann für kosmetische Reinigungsprodukte wie Schaumbäder, Duschbäder, Reinigungsschäume, Shampoos, für Produkte der Haarpflege sowie für Produkte zur Körper- und Hautpflege, wie Cremes, Lotionen, Deos etc. eingesetzt werden. Die Produkte können auch auf wässriger, wässrig-alkoholischer oder wässrig-glycolischer Basis formuliert werden, und beispielsweise Duftwässer, Gesichtswässer und After-Shaves umfassen. Grundsätzlich liegen hier keine Einschränkungen vor.

Als Komponente (A) der Stabilisator-Mischung fungieren Tocopherole oder Tocopherol-Derivate, die zu den sogenannten Biochinonen zählen. Tocopherole bestehen chemisch gesehen aus einem Chromanring und einer Phytolseitenkette. Erfindungsgemäß einsetzbar ist natürliches oder synthetisches Tocopherol, wobei natürliches Tocopherol bevorzugt ist. Natürliches Tocopherol ist ein Gemisch aus vier Isomeren, die sich nur in der Substitution am Chromanring unterscheiden, deren Phytolseitenkette jedoch immer die RRR-Konfiguration hat. Hierzu gehören α-, β-, γ-, δ- und ε-Tocopherol. Synthetisch hergestelltes Tocopherol weist dagegen auch ein Isomerengemisch in der Phytolseitenkette auf. Erfindungsgemäß besonders bevorzugt sind Tocopherol-Mischungen mit einem hohen Gehalt an γ- und δ-Isomeren. Natürliche Tocopherol-Mischungen können aus vielen Pflanzenölen gewonnen werden. Besonders reich an Tocopherolen sind die Samenöle von Soja, Weizen, Mais, Reis, Baumwolle, Luzerne und Nüssen. Erfindungsgemäß einsetzbar sind auch die Ester des Tocopherols, wie beispielsweise Tocopherylacetat, -succinat, -nicotinat und -poly(oxyethylen)succinat. Die Tocopherol-Komponente (A) oder das Gemisch aus Tocopherolen ist in den erfindungsgemäßen kosmetischen Mitteln in Konzentrationen von 0,005 - 5 Gew.-%, bevorzugt 0,01 - 2 Gew.-% und besonders bevorzugt 0,02 - 1 Gew.-%, jeweils bezogen auf das Gewicht des behälterfreien kosmetischen Mittels, enthalten.

Als Komponente (B) sind Komplexbildner enthalten. Erfindungsgemäß werden hierunter Verbindungen verstanden, die Metall-Kationen chelatartig komplexieren, insbesondere Calcium-, Eisen-, Nickel- und Kupfer-Ionen. Dem Fachmann sind eine Vielzahl solcher Verbindungen aus der einschlägigen Literatur bekannt. Für kosmetische Zwecke besonders geeignet sind Polycarbonsäuren und deren wasserlösliche Natrium-, Kalium-, Magnesium- und Ammoniumsalze, verschiedene Fruchtsäuren, Derivate von Aminosäuren, Aminopolycarbonsäuren, Phosphonsäuren: Erfindungsgemäß geeignet sind Hydroxycarbonsäuren und deren Salze, wie z. B. Citronensäure, Weinsäure, Äpfelsäure, Gluconsäure, Glucuronsäure, Galactarsäure, Phytinsäure, Polycarbonsäuren wie z. B. Bernsteinsäure, 1,2,3-Propantricarbonsäure, Cyclodextrine, β-Alanindiessigsäure und deren Salze, Dihydroxyethylglycinate, Dicarboxymethylalaninate, Iminodisuccinate, Tetrahydroxyethyl- und Tetrahydroxypropylethylendiamin.

Bevorzugt geeignet als Komplexbildner (B) sind die wasserlöslichen Salze von Aminopolycarbonsäuren, insbesondere die Natrium-, Kalium-, Ammonium-, Magnesium-, Calcium- und Triethanolaminsalze. Hierzu gehören beispielsweise Ethylendiamintetraessigsäure (EDTA) und deren Salze, z. B. Calcium-Dinatrium-EDTA, Diammonium-EDTA, Dinatrium- und Dikalium-EDTA, Triethanolamin-EDTA, Trinatrium- und Trikalium-EDTA, Tetranatrium- und Tetrakalium-EDTA, sowie Nitrilotriessigsäure, Hydroxyethylethylendiamintriessigsäure, Cyclohexandiamintetraessigsäure, Diethylentriaminpentaessigsäure, Lauroylethylendiamintriessigsäure und Ethylendiamindibernsteinsäure und die entsprechenden Salze. Erfindungsgemäß besonders bevorzugt als Komponente B ist Tetranatrium-EDTA und Trinatrium-EDTA, weiche zum Beispiel unter dem Namen Trilon^{®} B und Trilon^{®} A im Handel sind.

Der Komplexbildner oder das Gemisch aus Komplexbildnern (B) ist in den erfindungsgemäßen kosmetischen Mitteln üblicherweise in Konzentrationen von 0,01 - 3 Gew.-%, bevorzugt 0,02 - 2 Gew.%, besonders bevorzugt 0,05 - 1 Gew.%, jeweils bezogen auf das Gewicht des behälterfreien kosmetischen Mittels, enthalten.

Als Komponente (C) ist wenigstens ein Ester aus einer hydroxysubstituierten Bi- oder Tricarbonsäure und einem C₁₂-C₂₄-Fettsäuremono- und/oder -diglycerid enthalten. Erfindungsgemäß geeignet als hydroxysubstituierte Bi- oder Tricarbonsäuren der Ester (C) sind insbesondere Fruchtsäuren wie beispielsweise Weinsäure, Citronensäure, Äpfelsäure. Erfindungsgemäß bevorzugt sind die Citronensäureester (Citrate) von C₁₂-C₂₄-Fettsäuremono- und -diglyceriden.

Bei den Fettsäuremono- und -digtycerid-Komponenten der Ester (C) handelt es sich um bekannte Stoffe, die nach einschlägigen Verfahren der präparativen organischen Chemie hergestellt werden können. Beispielsweise werden die Citrate der Mono- und Diglyceride durch Umesterung der entsprechenden Triglyceride mit Glycerin und Citronensäure oder durch gezielte Veresterung von Fettsäuren und Citronensäure mit Glycerin erhalten. Die Abtrennung von nichtumgesetzten Ausgangsstoffen sowie die Anreicherung bestimmter Komponenten in den Gemischen erfolgt üblicherweise über eine Molekulardestillation. Als Esterkomponente bevorzugt geeignet sind die Citrate unverzweigter, gesättigter C₁₂-C₂₀-Fettsäuremono- und -diglyceride, und unter diesen insbesondere der C₁₂-C₁₈-Fettsäuremono- und -diglyceride. Explizite Beispiele für derartige Citrate sind Glycerylmonostearatcitrat, Glyceryldistearatcitrat, Glycerylmonopalmitatcitrat, Glycerytdipalmitatcitrat, Glycerylmonomyristatcitrat, Glycerytdimyristatcitrat, Glycerylmonolauratcitrat, Glyceryldilauratcitrat sowie Mischungen dieser Glyceridcitrate, insbesondere Mischungen aus Glycerylmonostearatcitrat, Glyceryldistearatcitrat, Glycerylmonopalmitatcitrat und Glyceryldipalmitatcitrat. Derartige Citrate sind zum Beispiel unter dem Namen IMWITOR^{®} 370 als Reinsubstanz oder in Form von Produktgemischen wie Controx^{®} KS und Controx^{®} VP erhältlich.

Der Ester (C) ist in den erfindungsgemäßen kosmetischen Mitteln in Konzentrationen von 0,005 - 1 Gew.-%, bevorzugt 0,01 - 1 Gew.-% und besonders bevorzugt 0,02 - 1 Gew.-%, jeweils bezogen auf das Gewicht des behälterfreien kosmetischen Mittels, enthalten.

Das bevorzugte Gewichtsverhältnis der Komponenten a : b : c beträgt 1 : (0,5 - 80) : (0,5 - 3), bezogen auf den Aktivsubstanzgehalt.

Als Stabilisator-Mischung besonders geeignet ist eine Kombination aus wenigstens einem Tocopherol und/oder Tocopherol-Derivat (A), einem Salz einer Ethylendiamintetraessigsäure (B) und einem Citronensäureester eines C₁₂-C₁₈-Fettsäuremono- und/oder -diglycerids (C). Kombinationen aus Trilon^{®} B und Controx^{®} KS können bevorzugt sein.

Als weitere obligatorische Komponente enthält das kosmetische Mittel Wasser, das in Mengen von 5 - 90 Gew.-%, bevorzugt 10 - 80 Gew.-% und besonders bevorzugt 20 - 70 Gew.-%, jeweils bezogen auf das Gewicht des behälterfreien kosmetischen Mittels, enthalten ist.

Das kosmetische Mittel des Produktes enthält vorzugsweise mindestens ein Tensid ausder Gruppe der Aniontenside, der Niotenside, der amphoteren, der zwitterionischen Tenside und/oder der Kationtenside.
Die Tenside sind in Mengen von 5 bis 40 Gew.-%, bevorzugt 5 bis 20 Gew.-%, jeweils bezogen auf das Gewicht des behälterfreien kosmetischen Mittels, enthalten.

Als anionische Tenside eignen sich in erfindungsgemäßen Mitteln alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium-, Magnesium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R¹-O-(CH₂-CH₂O)ₓ-CH₂-COOH, in der R¹ eine lineare Alkylgruppe.mit 10 bis 22 C-Atomen und x = 0 oder 1 bis 16 ist,
- Amidethercarboxylate der Formel [R²-NH(-CH₂-CH₂-O)ₙ-CH₂-COO]ₘZ, in der R² für einen linearen oder verzweigten, gesättigten oder ungesättigten Acylrest mit 2 bis 29 C-Atomen, n für ganze Zahlen von 1 bis 10, m für die Zahlen 1 oder 2 und Z für ein Kation aus der Gruppe der Alkali- oder Erdalkalimetalle steht,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und dialkylester mit 8 bis 18 C-Atomen in der Alkylgruppe und Sulfobemsteinsäuremonoalkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R³-O(-CH₂-CH₂O)_{X}-SO₃H, in der R³ eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Kokosmonoglyceridsulfate.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, sowie Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R⁴O-(S)ₓ. Diese unterstützen die Milde der erfindungsgemäßen Mittel, haben einen verdickenden Effekt und tragen zu einer verbesserten Solublisierung der Fettsäurepartialglyceride bei. Sie sind durch die folgenden Parameter gekennzeichnet:
Der Alkylrest R⁴ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.
Die erfindungsgemäß verwendbaren Alkylpolyglykoside können beispielsweise nur einen bestimmten Alkylrest R⁴ enthalten. Üblicherweise werden diese Verbindungen aber ausgehend von natürlichen Fetten und Ölen oder Mineralölen hergestellt. In diesem Fall liegen als Alkylreste R⁴ Mischungen entsprechend den Ausgangsverbindungen bzw. entsprechend der jeweiligen Aufarbeitung dieser Verbindungen vor.
Besonders bevorzugt sind solche Alkylpolyglykoside, bei denen R⁴
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein S können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.
Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.
Die Alkylglykoside können neben ihrer Tensidwirkung auch dazu dienen, die Fixierung der Duftkomponenten auf dem Haar und der Haut zu verbessern.
Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.
Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.
Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteitung kann bevorzugt sein.
Weiterhin können, insbesondere als Co-Tenside, zwitterionische Tenside verwendet werden. Als zwitterionische Tenside werden solche oberflächenaktiven Verbindungen bezeichnet, die im Molekül mindestens eine quartäre Ammoniumgruppe und mindestens eine -COO⁻- oder -SO₃⁻ -Gruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCl-Bezeichnung Cocamidopropyl Betaine bekannte Fettsäureamid-Derivat.
Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin Beispiele für die kationische Tenside, die vorzugweise in Haarbehandlungsmitteln verwendet werden, sind insbesondere quartäre Ammoniumverbindungen. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex^{®} vertriebenen Dialkylammoniummethosulfate und Methylhydroxyalkyldialkoyloxyalkylammoniummethosulfate und die entsprechenden Produkte der Dehyquart^{®}-Reihe, als kationische Tenside eingesetzt werden. Weitere erfindungsgemäß verwendbare kationische Tenside stellen die quaternisierten Proteinhydrolysate dar.

In einer bevorzugten Ausführungsform handelt es sich um einen Reinigungsschaum mit erfindungsgemäßer Stabilisator-Kombination, der als Tensidkomponenten eine Kombination aus einem Sulfobernsteinsäuremonoestersalz, einem Ampho- oder Betaintensid und einem Alkylpolyglycosid enthält.
In einer besonders bevorzugten Ausführungsform der Erfindung handelt es sich um ein Schaumreinigungsprodukt zur Körper- und Gesichtspflege, bestehend aus einem tensidhaltigen, die Stabilisator-Mischung enthaltenden Reinigungsmittel in einem transparenten oder semitransparenten, treibgasfreien Spendebehälter mit Schaumventil.

Spendebehälter mit Schaumdüse sind z. B. aus der US 3,709,437, der US 3,937,364, der US 5,635,469 oder WO 94/09345 A1 bekannt. In diesen Geräten wird die Reinigungsflüssigkeit entweder durch Druck auf die flexible Behälterwand (squeeze bottle) oder über eine manuell zu betätigende Pumpe zu dem Schaumventil befördert. In dem Schaumventil wird die Flüssigkeit dann mit Luft vermischt und als Schaum ausgetragen.

Abgesehen von dem als Stabilisator-Komponente eingesetzten Tocopherol enthält das kosmetische Mittel des Produktes in einer bevorzugten Ausführungsform weiterhin wenigstens einen pflegenden Wirkstoff, ausgewählt aus der Gruppe der Vitamine, Provitamine oder Vitaminvorstufen. Diese sind in den erfindungsgemäßen Mitteln in einer Menge von 0,1 - 10 Gew.%, vorzugsweise 0,2 - 5 Gew.-% und insbesondere 0,5 - 1 Gew.-%, jeweils bezogen auf das Gewicht des behälterfreien kosmetischen Mittels, enthalten. Dabei sind erfindungsgemäß solche Vitamine, Pro-Vitamine und Vitaminvorstufen bevorzugt, die üblicherweise den Gruppen A, B, C, F und H zugeordnet werden.

Zur Gruppe der als **Vitamin A** bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht.
Zur **Vitamin B-Gruppe** oder Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt, unter denen insbesondere das Nicotinsäureamid erfindungsgemäß bevorzugt ist.
- Vitamin B₅ (Pantothensäure und Panthenol). Im Rahmen dieser Gruppe wird bevorzugt das Panthenol eingesetzt. Erfindungsgemäß einsetzbare Derivate des Panthenols sind insbesondere die Ester und Ether des Panthenols, kationisch derivatisierte Panthenole sowie Pantolacton.
- Vitamin B₆ (Pyridoxin sowie Pyridoxamin und Pyridoxal).

Erfindungsgemäß geeignet sind auch **Vitamin C** (Ascorbinsäure) und dessen Ester, insbesondere Ascorbylpalmitat.

**Vitamin F.** Unter dem Begriff "Vitamin F" werden üblicherweise essentielle Fettsäuren, insbesondere Linolsäure, Linolensäure und Arachidonsäure, verstanden.
**Vitamin H.** Als Vitamin H wird die Verbindung (3a*S*,4*S*, 6a*R*)-2-Oxohexahydrothienol[3,4-*d*]-imidazol-4-valeriansäure bezeichnet, für die sich aber zwischenzeitlich der Trivialname Biotin durchgesetzt hat.

In einer weiteren bevorzugten Ausführungsform enthält das kosmetische Mittel des erfindungsgemäßen Produktes wenigstens einen Pflanzenextrakt oder ein Destillat aus Pflanzenbestandteilen. Pflanzenextrakte und -destillate steigern häufig die anderen Wirkstoffeigenschaften des Mittels. Die Pflanzenextrakte und -destillate können erfindungsgemäß sowohl in reiner als auch in verdünnter Form eingesetzt werden. Die erfindungsgemäßen Mittel können auch Mischungen aus mehreren verschiedenen Pflanzenextrakten und -destillaten enthalten. Die Pflanzenextrakte und -destillate sind üblicherweise in einer Menge von 0,01 - 5 Gew.-%, vorzugsweise 0,1 - 3 Gew.% und insbesondere 0,1 bis 2 Gew.-% Aktivsubstanz, jeweils bezogen auf das Gewicht des behälterfreien kosmetischen Mittels, enthalten.
Üblicherweise werden diese Extrakte und -destillate durch Extraktion der gesamten Pflanze bzw. durch Wasserdampfdestillation gewonnen. Es kann aber in einzelnen Fällen auch bevorzugt sein, die Extrakte und -destillate ausschließlich aus Blüten und/oder Blättern der Pflanze herzustellen.
Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.
Erfindungsgemäß sind vor allem die Extrakte und Destillate aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt. Erfindungsgemäß bevorzugt sind Mandelextrakte und Destillate aus Hamamelis und Salbei.
Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Bevorzugt enthalten die erfindungsgemäßen Mittel weiterhin mindestens ein organisches Verdickungsmittel. Solche Verdickungsmittel sind beispielsweise Verdickungsmittel wie Agar-Agar, Guar-Gum, Alginate, Celluloseether, Gelatine, Pektine und/oder Xanthan-Gum. Ethoxilierte Fettalkohole, insbesondere solche mit eingeschränkter Homologenverteilung, wie sie beispielsweise als Handelsprodukt unter der Bezeichnung Arlypon^{®}F (Henkel) auf dem Markt sind, alkoxylierte Methylglucosidester, wie das Handelsprodukt Glucamate^{®} DOE 120 (Amerchol), und ethoxylierte Propylenglykolester, wie das Handelsprodukt Antil^{®} 141 (Goldschmidt) können bevorzugte organische Verdickungsmittel sein.

Als konditionierende Wirkstoffe geeignete Silikonöle und Silikon-Gums sind insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200 und DC 1401 vertriebenen Produkte sowie das Handelsprodukt Fancorsil^{®} LIM-1. Ein geeignetes anionisches Silikonöl ist das Produkt Dow Corning^{®}1784.

Bevorzugt enthaltene pflanzliche Öle und Wachse sind Nachtkerzenöl, Jojobaöl, Sonnenblumenöl, Orangenöl, Mandelöl, Weizenkeimöl und Pfirsichkernöl. Nachtkerzenöl ist besonders bevorzugt.

Insbesondere für die Formulierung sehr milder kosmetischer Zubereitungen hat es sich weiterhin als vorteilhaft erwiesen, wenn die Menge an gelösten anorganischen Salzen auf weniger als 2 Gew.-%, insbesondere weniger als 0,5 Gew.-% begrenzt wird. Dabei ist auch zu beachten, daß solche Salze nicht nur z.B. zur Einstellung der Viskosität zugegeben werden, sondern auch durch andere Wirkstoffe, insbesondere Tenside, eingebracht werden können.

Weitere übliche Bestandteile für die erfindungsgemäßen Mittel sind:
- nichtionische Polymere wie beispielsweise Vinylpyrrolidon/Vinylacrylat-Copolymere, Polyvinylpyrrolidon und Vinylpyrrolidon/Vinylacetat-Copolymere,
- anionische Polymere, wie Polyacryl- und Polymethacrylsäuren, deren Salze, deren Copolymere mit Acrylsäure- und Methacrylsäureestern und -amiden und deren Derivate, die durch Kreuzvemetzung mit polyfunktionellen Agentien erhalten werden,
   Polyoxycarbonsäuren, wie Polyketo- und Polyaldehydocarbonsäuren und deren Salze,
   sowie Polymere und Copolymere der Crotonsäure mit Estern und Amiden der Acryl- und der Methacrylsäure, wie Vinylacetat-Crotonsäure- und Vinylacetat-Vinylpropionat-Crotonsäure-Copolymere,
- Strukturanten wie Glucose und Maleinsäure,
- haarkonditionierende Verbindungen wie Phospholipide, beispielsweise Sojalecithin, Ei-Lecithin und Kephaline,
- Parfümöle, insbesondere solche mit der Duftnote einer Frucht, wie beispielsweise von Apfel, Birne, Erdbeere, Pfirsich, Aprikose, Ananas, Banane, Kirsche, Kiwi, Mango, Kokos, Mandel, Grapefruit, Maracuja, Mandarine und Melone, oder der Duftnote eines Genußmittels, wie beispielsweise von Tabak, Cola, Kaugummi, Guarana, Schokolade, Kakao, Vanille, Sarsaparilla, Pfefferminze und Rum.
- Dimethylisosorbid und Cyclodextrine,
- Lösungsvermittler, wie Ethanol, Isopropanol, Ethylenglykol, Propylenglykol, Glycerin, Diethylenglykol und ethoxylierte Triglyceride,
- Farbstoffe,
- Antischuppenwirkstoffe wie Climbazol, Piroctone Olamine und Zink Omadine,
- Wirkstoffe wie Bisabolol und Allantoin,
- Lichtschutzmittel,
- Konsistenzgeber wie Zuckerester, Polyolester oder Polyolalkylether,
- Fette und Wachse, wie Walrat, Bienenwachs, Montanwachs, Paraffine, Ester, Glyceride und Fettalkohole,
- Fettsäurealkanolamide,
- Quell- und Penetrationsstoffe wie PCA, Glycerin, Propylenglykolmonoethylether, Carbonate, Hydrogencarbonate, Guanidine, Harnstoffe sowie primäre, sekundäre und tertiäre Phosphate,
- Trübungsmittel wie Latex oder Styrol/Acrylamid-Copolymere,
- Perlglanzmittel wie Ethylenglykolmono- und -distearat oder PEG-3-distearat,
- direktziehende Farbstoffe
- Treibmittel wie Propan-Butan-Gemische, N₂O, Dimethylether, CO₂ und Luft sowie
- weitere Antioxidantien,
- Bitterstoffe, wie beispielsweise Denatonium Benzoate,
- Konservierungsmittel.

Die nachfolgenden Beispiele sollen den Erfindungsgegenstand näher erläutern, ohne ihn hierauf zu beschränken.

### Erfindungsgemäße Beispiele

Die Mengenangaben in nachfolgenden Beispielen geben, soweit nicht anders vermerkt, die Gew.-% Aktivsubstanz (AS), bezogen auf das Gewicht des behälterfreien kosmetischen Mittels, an.

### Reinigungschaum (Beispiel 1)

| **Rohstoff** | **Gew.-% AS** |
|---|---|
| Na-Benzoat | 0,4 |
| Texapon^{®} SB 3 | 12 |
| Tego Betain^{®} 810 | 2 |
| Plantacare^{®} 2000 | 2,5 |
| Cetiol^{®} HE | 2 |
| Parfum | 0,3 |
| Sorbitol | 0,7 |
| Glycerin | 0,86 |
| Controx^{®} KS | 0,05 |
| Trilon^{®} B | 0,1 |
| Farbstoff Cl 42090 | 0,0001 |
| Citronensäure | 0,4 |
| Mandelextrakt, ölhaltig | 1,0 |
| Hamamelis Destillat | 3,0 |
| Nachtkerzenöl | 0,3 |
| d-Panthenol | 0,5 |
| Wasser demin. | ad 100 |

130 g der Zusammensetzung wurden in einen transparenten oder semitransparenten 150-ml Polyethylen-Behälter vom Typ Airfoamer F2 (Hersteller: Fa. Airspray International B.V.) eingefüllt.

### Duschbad (Beispiel 2)

| **Rohstoff** | **Gew.-% AS** |
|---|---|
| Texapon^{®} NSO | 8,00 |
| Texapon^{®} SB3 | 4,00 |
| Tego Betain^{®} L7 | 2,0 |
| Cetiol^{®} HE | 3,0 |
| Monomuls^{®} 90 L12 | 2,0 |
| Nutrilan^{®} H | 1,0 |
| Cutina^{®} AGS | 1,0 |
| Parfumöl | 1,0 |
| Konservierungstoffe | 0,4 |
| Citronensäure | q.s. |
| Farbstoff Cl 42090 | 0,0001 |
| Farbstoff Cl 47005 | 0,0001 |
| Controx^{®} KS | 0,05 |
| Trilon^{®} B | 0,1 |
| Grüner Tee Destillat | 3,0 |
| Wasser | ad 100 |

130 g der Zusammensetzung wurden in einen transparenten oder semitransparenten 150-ml Polyethylen-Behälter vom Typ Airfoamer F2 (Hersteller: Fa. Airspray International B.V.) eingefüllt.

### Gesichtswasser (Beispiel 3)

| **Rohstoffe** | **Gew.-% AS** |
|---|---|
| Ethylalkohol | 15,00 |
| Sorbitol | 5,00 |
| Hamamelis-Destillat | 5,00 |
| d-Panthenol | 0,20 |
| Campher | 0,05 |
| Lösungsvermittler | 1 |
| Farbstoff CI 16035 | 0,00008 |
| Controx^{®} KS | 0,05 |
| Trilon^{®} B | 0,1 |
| Wasser | ad 100 |

130 g der Zusammensetzung wurden in einen transparenten 150-ml Glas-Behälter eingefüllt.

### Mildes Shampoo (Beispiel 4)

| **Rohstoff** | **Gew.-%AS** |
|---|---|
| Texapon^{®} NSO | 10,0 |
| Akypo^{®} RLM 100 | 2,5 |
| Miranol^{®} C2M | 4,0 |
| Tween 20 | 3,0 |
| Genapol^{®} TS | 2,0 |
| Farbstoff Cl 42090 | 0,00007 |
| Farbstoff Cl 17200 | 0,00005 |
| Controx^{®} KS | 0,05 |
| Trilon^{®} B | 0,1 |
| Wasser | ad 100 |

130 g der Zusammensetzung wurden in einen transparenten oder semitransparenten 150-ml Polyethylen-Behälter vom Typ Airfoamer F2 (Hersteller: Fa. Airspray International B.V.) eingefüllt.

### Stabilitätstests gegenüber Licht

### Reinigungschaum

| | **V1** | **V2** | **V3** | **V4** | **V5** | **Erfindungsgemäß (Beispiel 1)** |
|---|---|---|---|---|---|---|
| **Rohstoff** | **%AS** | **%AS** | **%AS** | **%AS** | **%AS** | **%AS** |
| Na-Benzoat | 0,4 | 0,4 | 0,4 | 0,4 | 0,4 | 0,4 |
| Texapon^{®} SB 3 | 12 | 12 | 12 | 12 | 12 | 12 |
| Tego Betain^{®} 810 | 2 | 2 | 2 | 2 | 2 | 2 |
| Plantacare^{®} 2000 UP | 2,5 | 2,5 | 2,5 | 2,5 | 2,5 | 2,5 |
| Cetiol^{®} HE | 2 | 2 | 2 | 2 | 2 | 2 |
| Parfum | 0,3 | 0,3 | 0,3 | 0,3 | 0,3 | 0,3 |
| Sorbitol | 0,7 | 0,7 | 0,7 | 0,7 | 0,7 | 0,7 |
| Glycerin | 0,86 | 0,86 | 0,86 | 0,86 | 0,86 | 0,86 |
| Copherol^{®} F-1300 | - | - | - | 0,03 | - | - |
| Controx^{®} KS | - | - | 0,05 | - | 0,1 | 0,05 |
| Trilon^{®} B | - | 0,1 | | 0,1 | - | 0,1 |
| Farbstoff Cl 42090 | 0,0001 | 0,0001 | 0,0001 | 0,0001 | 0,0001 | 0,0001 |
| Citronensäure | 0,4 | 0,4 | 0,4 | 0,4 | 0,4 | 0,4 |
| Mandelextrakt ölhaltig | 1,0 | 1,0 | 1,0 | 1,0 | 1,0 | 1,0 |
| Hamamelis Destillat | 3,0 | 3,0 | 3,0 | 3,0 | 3,0 | 3,0 |
| Nachtkerzenöl | 0,3 | 0,3 | 0,3 | 0,3 | 0,3 | 0,3 |
| d-Panthenoi | 0,5 | 0,5 | 0,5 | 0,5 | 0,5 | 0,5 |
| Wasser demin. | ad 100 | ad 100 | ad 100 | ad 100 | ad 100 | ad 100 |

Die Reinigungsschäume V1-V5 (nicht-erfindungsgemäße Vergleichsbeispiele) und der erfindungsgemäße Reinigungsschaum wurden in transparente und semitransparente Polyethylen-Flaschen abgefüllt, und die Lichtstabilität im Sonnenlichtsimulationsrriodell für eine Zeitdauer von 1 - 9 Stunden sowie bei Lagerung in einem nach Süden ausgerichteten Fenster über eine Zeitspanne von 2 Wochen untersucht.

### 1. Lichtstabilität im Sonnenlichtsimulationsmodell

Ver-*I* Entfärbungen der Rezepturen nach 1, 2, 5, 8 und 9 Stunden sind in Skalenwerten von 1 - 6 wiedergegeben, die von Testpersonen ermittelt wurden (1 = nicht entfärbt; 6 = vollständig entfärbt).

| Rezeptur | Nach 1h | Nach 2h | Nach 5h | Nach 8h | Nach 9h |
|---|---|---|---|---|---|
| **V1** | 1 | 6 | 6 | 6 | 6 |
| **V2** | 1 | 1 | 4 | 5 | 6 |
| **V3** | 1 | 6 | 6 | 6 | 6 |
| **V4** | 1 | 1 | 4 | 5 | 6 |
| **V5** | 1 | 6 | 6 | 6 | 6 |
| **Beispiel 1** | 1 | 1 | 2 - 3 | 3 | 3 - 4 |

### 2. Lichtstabilität bei Lagerung der Rezepturen am Fenster, Südseite

Ver-/ Entfärbungen der Rezepturen nach 2-wöchiger Lagerung sind in Skalenwerten von 1-6 wiedergegeben, die von Testpersonen ermittelt wurden (1 = nicht entfärbt; 6 = vollständig entfärbt).

| **Rezeptur** | **V1** | **V2** | **V3** | **V4** | **V5** | **(Beispiel 1)** |
|---|---|---|---|---|---|---|
| Ver-/ Entfärbung | 5 | 5 | 5 | 4 | 5 | 2 |

Die Stabilität der Reinigungsschäume gegenüber Licht ist somit am stärksten für die erfindungsgemäße Stabilisator-Kombination aus drei Komponenten. Die in den Vergleichsversuchen verwendeten Stabilisator-Komponenten führen zu stärkeren Ent- bzw. Verfärbungen.

### 3. Einfluß von UV-Absorbern im Produktbehälter oder im kosmetischen Mittel

Folgende Kombinationen wurden im Sonnenlichtsimulationsmodell (Zeitintervalle wie unter 1 beschrieben) und bei 2-wöchiger Lagerung an einem nach Süden ausgerichteten Fenster untersucht.
a) Reinigungsschaum V1 in Behälter ohne UV-Filter
b) Reinigungsschaum V1 mit UV-Filter (2,2',4,4'-Tetrahydroxybenzophenon) in Behälter ohne UV-Filter
c) Reinigungsschaum V1 in Behälter mit UV-Filter (Benzotriazol)
d) Reinigungsschaum V1 mit UV-Filter (2,2',4,4'-Tetrahydroxybenzophenon) in Behälter mit UV-Filter (Benzotriazol)
e) Erfindungsgemäßer Reinigungsschaum (Beispiel 1) in Behälter mit UV-Filter (Benzotriazol)

In beiden Tests ergab sich die Stabilitätsreihe a) < b) < c) = d) < e, die belegt, daß weder UV-Filter im Behältermaterial noch im Produkt eine ausreichende Lichtstabilität gewährleisten. Erst bei zusätzlicher Verwendung der erfindungsgemäßen Stabilisator-Kombination erreicht man in transparenten oder semitransparenten Behältern eine ausreichende Stabilität gegen Lichteinwirkung.

### 4. Liste der verwendeten Rohstoffe:

1) Cetiol^{®} HE
   INCl: PEG-7 Glyceryl cocoate
   Hersteller: Cognis Deutschland GmbH (Henkel)
2) Controx^{®} KS
   INCl: Tocopherol, Hydrogenated Palm Glycerides Citrate
   Tocopherolgehalt mindestens 56 %
   Hersteller: Cognis Deutschland GmbH (Grünau)
3) Cutina^{®} AGS
   INCl: Glycol Distearate
   Hersteller: Cognis Deutschland GmbH (Henkel)
4) Grüner Tee, Destillat (1 - 5 Gew.-% Aktivsubstanz)
   Hersteller: Cosmetochem AG, Schweiz
5) Hamamelis-Destillat (1 - 5 Gew.-% Aktivsubstanz)
   Hersteller: Cosmetochem AG, Schweiz
6) Mandelextrakt, ölhaltig (10 - 25 Gew.-% Aktivsubstanz)
   Hersteller: Grau Aromatics
7) Monomuls^{®} 90 L12
   INCI: Glyceryl Laurate
   Hersteller: Cognis Deutschland GmbH (Grünau)
8) Nutrilan^{®} H
   Eiweiß-Hydrolysat, Natrium-Salz
   Hersteller: Cognis Deutschland GmbH (Grünau)
9) Plantacare^{®} 2000 UP
   INCI: Decyl Glucoside, Aqua (Water)
   Hersteller: Cognis Deutschland GmbH (Henkel)
10) Tego Betain^{®} 810
   INCl: Capryl/Capramidopropyl Betaine
   Hersteller: Tego Cosmetics (Goldschmidt-Rewo)
11) Tego Betain^{®} L7
   INCI: Cocamidopropyl Betaine
   Hersteller: Tego Cosmetics (Goldschmidt-Rewo)
12) Texapon^{®} NSO
   INCI: Sodium Laureth Sulfate
   Hersteller: Cognis France S.A. (Sidobre Sinnova)
13) Texapon^{®} SB 3
   INCI: Aqua (Water), Disodium Laureth Sulfosuccinate, Citric Acid
   Hersteller: Cognis Deutschland GmbH (Henkel)
14) Trilon^{®} B, flüssig
   INCl: Tetrasodium EDTA
   Hersteller: BASF
15) Copherol^{®} F 1300
   INCI: Tocopherol
   Hersteller: Cognis Corporation
   Aktivsubstanz mindestens 85,6% (min)
16) Akypo ^{®} RLM 100
   INCI: Laureth-11 Carboxylic Acid
   Hersteller: KAO
17) Miranol ^{®} C2M
   INCl: Disodium Cocoamphodiacetate
   Hersteller: Rhodia
18) Genapol ^{®} TS
   INCI: PEG-3 Distearate
   Hersteller: Clariant
19) Tween^{®} 20
   INCl: Polysorbate 20
   Hersteller: Uniquema (ICI-Surfactants)

## Patentansprüche

1. Wasserhaltiges kosmetisches Mittel, enthaltend eine Stabilisator-Mischung aus
a) wenigstens einem Tocopherol und/oder Tocopherol-Derivat (A) und
b) wenigstens einem Komplexbildner (B) und
c) 0.005 bis 1 Gew.-%, bezogen auf das Gewicht des kosmetischen Mittels, eines Esters (C) aus einer hydroxysubstituierten Bi- oder Tricarbonsäure und einem C₁₂-C₂₄-Fettsäuremono- und/oder-digtycerid.

2. Wasserhaltiges kosmetisches Mittel gemäß Anspruch 1, **dadurch gekennzeichnet, daß** der Komplexbildner (B) ausgewählt ist aus den wasserlöslichen Salzen der Aminopolycarbonsäuren.

3. Wasserhaltiges kosmetisches Mittel gemäß Anspruch 1 oder 2. **dadurch gekennzeichnet daß** die hydroxysubstituierte Tricarbonsäure des Esters (C) Citronensäure ist.

4. Wasserhaltiges kosmetisches Mittel gemäß einem der Ansprüche 1 bis 3, **dadurch gekennzeichnet, daß** das Gewichtsverhältnis der Komponenten a : b : c = 1 : (0.5 - 80) : (0,5 - 3) beträgt.

5. Wasserhaltiges kosmetisches Mittel gemäß einem der Ansprüche 1 bis 4. **dadurch gekennzeichnet, daß** die Stabilisator-Mischung wenigstens
a) ein Tocopherol und/oder Tocopherol-Derivat (A) und
b) ein Salz einer Ethylendiamintetraessigsäure (B) und
c) 0,005 bis 1 Gew.-%, bezogen auf das Gewicht des behälterfreien kosmetischen Mittels, eines Citronensäureesters eines C₁₂-C₁₆-Fettsäuremono- und/oder -diglycerids (C)
enthält.

6. Wasserhaltiges kosmetisches Mittel gemäß einem der Ansprüche 1 bis 5. **dadurch gekennzeichnet, daß** mindestens ein Tensid aus der Gruppe der Aniontenside, der Niotenside, der amphoteren und zwitterionischen Tenside oder der Kationtenside enthalten ist.

7. Wasserhaltiges kosmetisches Mittel gemäß einem der Ansprüche 1 bis 6, **dadurch gekennzeichnet, daß** wenigstens ein pflegender Wirkstoff, ausgewählt aus der Gruppe der Vitamine. Provitamine oder Vitaminvorstufen, enthalten ist.

8. Wasserhaltiges kosmetisches Mittel gemäß einem der Ansprüche 1 bis 7. **dadurch gekennzeichnet, daß** wenigstens ein Extrakt oder ein Destillat von Pflanzenbestandteilen enthalten ist.

9. Kosmetisches Produkt, bestehend aus einem wasserhaltigen kosmetischen Mittel gemäß einem der Ansprüche 1 - 8 und einem transparenten oder semitransparenten Behälter.

10. Verwendung einer Kombination aus
a) wenigstens einem Tocopherol und/oder Tocopherol-Derivat (A) und
b) wenigstens eines Komplexbildner (B) und
c) 0,005 bis 1 Gew.-%, bezogen auf das Gewicht des kosmetischen Mittels, eines Esters (C) aus einer hydroxysubstituierten Bi- oder Tricarbonsäure und einem C₁₂-C₂₄-Fettsäuremono- und/oder -diglycerid
zur Farb- und Geruchsstabilisierung kosmetischer Mittel in transparenten oder semitransparenten Produktbehältern, insbesondere gegenüber der Einwirkung von Licht.

11. Verfahren zur Farb- und Geruchsstabilisierung kosmetischer Mittel in transparenten oder semitransparenten Behältern, **dadurch gekennzeichnet, daß** man den Mitteln eine Stabitisator-Mischung aus
a) wenigstens einem Tocopherol und/oder Tocopherol-Derivat (A) und
b) wenigstens einem Komplexbildner (B) und
c) 0.005 - 1 Gew.-%, bezogen auf das Gewicht des kosmetischen Mittels, eines Esters (C) aus einer hydroxysubstituierten Bi- oder Tricarbonsäure und einem C₁₂-C₂₄-Fettsäuremono- und/oder -diglycerid
zusetzt.

## Claims

1. Hydrous cosmetic agent comprising a stabilizer mixture of
a) at least one tocopherol and/or tocopherol derivative (A) and
b) at least one complexing agent (B) and
c) 0.005 to 1% by weight, based on the weight of the cosmetic agent, of an ester (C) of a hydroxy-substituted bi- or tricarboxylic acid and a C₁₂-C₂₄-fatty acid mono- and/or diglyceride.

2. Hydrous cosmetic agent according to Claim 1, **characterized in that** the complexing agent (B) is chosen from the water-soluble salts of aminopolycarboxylic acids.

3. Hydrous cosmetic agent according to Claim 1 or 2, **characterized in that** the hydroxy-substituted tricarboxylic acid of ester (C) is citric acid.

4. Hydrous cosmetic agent according to one of Claims 1 to 3, **characterized in that** the weight ratio of components a:b:c = 1:(0.5-80):(0.5-3).

5. Hydrous cosmetic agent according to one of Claims 1 to 4, **characterized in that** the stabilizer mixture comprises at least
a) one tocopherol and/or tocopherol derivative (A) and
b) a salt of an ethylenediaminetetraacetic acid (B) and
c) 0.005 to 1% by weight, based on the weight of the container-free cosmetic agent, of a citric ester of a C₁₂-C₁₈-fatty acid mono- and/or diglyceride (C).

6. Hydrous cosmetic agent according to one of Claims 1 to 5, **characterized in that** at least one surfactant from the group of anionic surfactants, nonionic surfactants, amphoteric and zwitterionic surfactants or cationic surfactants is present.

7. Hydrous cosmetic agent according to one of Claims 1 to 6, **characterized in that** at least one care active ingredient chosen from the group of vitamins, provitamins or vitamin precursors is present.

8. Hydrous cosmetic agent according to one of Claims 1 to 7, **characterized in that** at least one extract or one distillate of plant constituents is present.

9. Cosmetic product consisting of a hydrous cosmetic agent according to one of Claims 1-8 and a transparent or semitransparent container.

10. Use of a combination of
a) at least one tocopherol and/or tocopherol derivative (A) and
b) at least one complexing agent (B) and
c) 0.005 to 1% by weight, based on the weight of the cosmetic agent, of an ester (C) of a hydroxy-substituted bi- or tricarboxylic acid and a C₁₂-C₂₄-fatty acid mono- and/or diglyceride for stabilizing the colour and odour of cosmetic agents in transparent or semitransparent product containers, in particular against the effect of light.

11. Process for stabilizing the colour and odour of cosmetic agents in transparent or semitransparent containers, **characterized in that** a stabilizer mixture of
a) at least one tocopherol and/or tocopherol derivative (A) and
b) at least one complexing agent (B) and
c) 0.005 to 1% by weight, based on the weight of the cosmetic agent, of an ester (C) of a hydroxy-substituted bi- or tricarboxylic acid and a C₁₂-C₂₄-fatty acid mono- and/or diglyceride
is added to the agents.

## Revendications

1. Agent cosmétique aqueux contenant un mélange de stabilisateurs constitué par :
a) au moins un tocophérol et/ou un dérivé de tocophérol (A) ; et
b) au moins un formateur de complexes (B) ; et
c) à concurrence de 0,005 à 1 % en poids, rapporté au poids de l'agent cosmétique, un ester (C) d'un acide dicarboxylique ou tricarboxylique substitué par un ou plusieurs groupes hydroxyle et d'un monoglycéride et/ou diglycéride d'acide gras en C₁₂-C₂₄.

2. Agent cosmétique aqueux selon la revendication 1, **caractérisé en ce que** le formateur de complexes (B) est choisi parmi le groupe comprenant des sels hydrosolubles d'acides aminopolycarboxyliques.

3. Agent cosmétique aqueux selon la revendication 1 ou 2, **caractérisé en ce que** l'acide tricarboxylique de l'ester (C) substitué par un ou plusieurs groupes hydroxyle est l'acide citrique.

4. Agent cosmétique aqueux selon l'une quelconque des revendications 1 à 3, **caractérisé en ce que** le rapport pondéral des composants a : b : c s'élève à 1 : (0,5 - 80) : (0,5 - 3).

5. Agent cosmétique aqueux selon l'une quelconque des revendications 1 à 4, **caractérisé en ce que** le mélange de stabilisateurs contient au moins :
a) un tocophérol et/ou un dérivé de tocophérol (A) ; et
b) un sel d'acide éthylènediaminetétracétique (B) ; et
c) à concurrence de 0,005 à 1 % en poids, rapporté au poids de l'agent cosmétique exempt de récipient, un ester d'acide citrique d'un monoglycéride et/ou diglycéride d'acide gras en C₁₂-C₁₈ (C).

6. Agent cosmétique aqueux selon l'une quelconque des revendications 1 à 5, **caractérisé en ce qu'**il contient au moins un agent tensioactif choisi parmi le groupe comprenant des agents tensioactifs anioniques, des agents tensioactifs non ioniques, des agents tensioactifs amphotères et zwitterioniques ou des agents tensioactifs cationiques.

7. Agent cosmétique aqueux selon l'une quelconque des revendications 1 à 6, **caractérisé en ce que** qu'il contient au moins une substance active d'entretien choisie parmi le groupe comprenant des vitamines, des provitamines ou des précurseurs de vitamines.

8. Agent cosmétique aqueux selon l'une quelconque des revendications 1 à 7, **caractérisé en ce que** qu'il contient au moins un extrait ou un distillat de constituants végétaux.

9. Produit cosmétique constitué par un agent cosmétique aqueux selon l'une quelconque des revendications 1 à 8 et par un récipient transparent ou semi-transparent.

10. Utilisation d'une combinaison de
a) au moins un tocophérol et/ou un dérivé de tocophérol (A) ; et
b) au moins un formateur de complexes (B) ; et
c) à concurrence de 0,005 à 1 % en poids, rapporté au poids de l'agent cosmétique, un ester (C) d'un acide dicarboxylique ou tricarboxylique substitué par un ou plusieurs groupes hydroxyle et d'un monoglycéride et/ou diglycéride d'acide gras en C₁₂-C₂₄
pour la stabilisation de la couleur et de l'odeur d'agents cosmétiques dans des récipients de produits transparents ou semi-transparents, en particulier contre l'effet de la lumière.

11. Procédé pour la stabilisation de la couleur et de l'odeur d'agents cosmétiques dans des récipients transparents ou semi-transparents, **caractérisé en ce qu'**on ajoute aux agents, un mélange de stabilisateurs constitué par
a) au moins un tocophérol et/ou un dérivé de tocophérol (A) ; et
b) au moins un formateur de complexes (B) ; et
c) à concurrence de 0,005 à 1 % en poids, rapporté au poids de l'agent cosmétique, un ester (C) d'un acide dicarboxylique ou tricarboxylique substitué par un ou plusieurs groupes hydroxyle et d'un monoglycéride et/ou diglycéride d'acide gras en C₁₂-C₂₄.
